# EUROPEAN PATENT SPECIFICATION

(11) **EP 3 202 377 B1**
(45) Date of publication and mention of the grant of the patent: **22.04.2020**
(21) Application number: 17154590.8
(22) Date of filing: 03.02.2017
(51) Int. Cl.: A61F 5/01, A61N 1/04, A61F 5/058

(54) **BUILDING METHOD OF AN IMMOBILIZATION DEVICE APPLICABLE TO ONE BODY SEGMENT OF A PATIENT**
EIN HERSTELLUNGSVERFAHREN EINES IMMOBILISIERUNGSGERÄTES FÜR EIN KÖRPER SEGMENT EINES PATIENTEN
MÉTHODE DE CONSTRUCTION D'UN DISPOSITIF D'IMMOBILISATION APPLICABLE A UN SEGMENT DE CORPS D'UN PATIENT

(30) Priority: 04.02.2016 IT UB20160286
(43) Date of publication of application: 09.08.2017
(73) Proprietor: Ranaldo, Davide, 35031 Abano Terme (PD) (IT); Lazzaro, Jacopo, 35139 Padova (PD) (IT)
(72) Inventor: RANALDO, Davide, 35031 Abano Terme (PD) (IT); LAZZARO, Jacopo, 35139 Padova (PD) (IT)
(74) Representative: Trentin, Michele

(56) References cited:
- EP-A2- 2 671 544
- WO-A1-82/00951
- WO-A2-2011/045614
- US-A1- 2014 163 444

## Description

### Field of invention

The present invention is generally applicable to the medical devices sector, particularly to the devices used in orthopedics and trauma.

The present invention relates to a building method of an immobilization device applicable to at least one body segment of a patient for at least partially immobilize it.

### Background of the invention

In the orthopedics medical discipline often happens that it is necessary to apply an at least partially rigid tutor or an immobilization cast to a body segment of the patient in order to cure him.

This usually happens in presence of fractures to limbs which, once recomposed, it need to temporarily immobilize the limb to allow its healing. Such example, however, is not the only case for the application of rigid or at least partially rigid tutors to a body segment of the body of a patient. An example are sprain traumas where the affected body segment must be immobilized in order to allow a correct and rapid recovery.

In the known technic usually proceeds by applying a cast or a standard preformed tutor on the affected area, but this solution presents several known drawbacks.

First of all, in case of a medical inspection it is necessary to remove the cast device and proceed to apply a new immobilization tutor at the end of the inspection. In addition, the cast is handmade and left to the expertise of the doctor, who can execute it too tight and with a non-optimal shaping.

In several cases, moreover, there is no need for a rigid tutor, but one at least partially flexible in order to simply aid to reinforce the mechanical seal of the affected body segment whilst leaving an at least partial mobility.

Therefore, they are known flexible tutors usually constituted by more or less thick elastic material, and usually reinforced with metallic sticks. In this case the doctor's inspection is made easier, but this solution is not suitable in case there is the need to have a certain rigidity in the body segment to heal.

In addition, it is often necessary to rigid only some areas of the segment, while other can be only partially rigid and others can be left flexible. Such type of inspection, which varies case by case, is substantially impossible to achieve with a cast, and very difficult and expensive, as well as inaccurate, with a flexible tutor reinforced with sticks.

Therefore, there are known rigid or semi rigid tutors made using 3D printing. In those cases it proceeds with a three-dimensional scan of the affected body segment, which is then elaborated and subsequently printed with the 3D printer.

These tutors are made of at least two facing shells closable by closures that allow its opening for inspection.

If these allows to obtain a perfectly adapted tutor for the patient even if not handmade, it does not, however, allows a precise control of which areas are to leave rigid, partially rigid of flexible.

It is also known that, often, in order to facilitate the healing process, the use of the tutor should be, as much as possible, accompanied by the use of electromagnetic therapy treatments, such as TENS, Magnetotherapy, TECARtherapy, or similar.

However, the abovementioned tutors hinder these treatments, either because they constitute a partial shield to the generated magnetic fields, or because they do not allow the application in loco of the specific electrodes. This results in the postponing of these treatments to the rehabilitation process to the affected body segment that takes place after the final removal of the tutor.

Document EP 2 671 544 A2 is also known. It teaches to use a 3D printer to execute (with a single nozzle) the core of an immobilization device to couple to a cover provided with electrodes and electrical connections for rehabilitation treatments. As a consequence, the electrodes end the electrical connection are note uniform with the core of the immobilization device.

### Summary of the invention

The invention is defined in the appended claims. Aspects, embodiments and examples disclosed herein which do not fall within the scope of the appended claims do not form part of the invention, and are merely provided for illustrative purposes.

Object of the invention is to at least partially overcome the drawbacks abovementioned, providing immobilization device of a body segment of a patient for at least partially immobilize it that has the abovementioned different rigidity areas.

Another object of the invention is to provide and immobilization device of a body segment of a patient for at least partially immobilize it that allows to made easily and accurately the abovementioned different rigidity areas.

Another object is that the said device allows to easily apply different therapies, such as TENS, Magnetotherapy or TECARtherapy.

Another object of the invention is that the said device allows these treatments without its removal.

In other words, an object of the invention is to provide an immobilization device that does not need its removal in order to optimally perform rehabilitative electromagnetic treatments.

These objects and other that will appear more clearly in the following, are achieved by an immobilization device of at least one body segment of a patient for at least partially immobilize it obtained according to the method of the following claims, which are to be considered an integral part of the present patent document.

Particularly, this method comprises a detection step of the volumetric dimensions of the patient's body segment to be at least partially immobilized with the device.

There is then a setting step of the shaping of the immobilization device. This shaping must necessarily consider not only the body segment volume to immobilize, but also of its possible swelling. The same shaping must also be suited for the position to be kept by the body segment, and it should take account of applicable grips, that is the differentiation in rigidity area by area that the tutor must have.

There is then the subsequent printing step of the immobilization device through a 3D printer. The 3D printed comprises, particularly, at least one pair of nozzles for the emission of materials having different physical characteristics between them, so as to vary, area by area, the physical characteristics of the immobilization device.

In other words, advantageously, the immobilization device is obtained through a 3D printer. This renders it easily customizable and adaptable to the patient and its needs. At the same time, it results from a calculation planning, and thereby compensating any defective expertise of the operators in the execution of these devices.

With reference to the use of different materials, it is evident that, advantageously, it allows obtaining tutors with different characteristics between them and, particularly, tutors with different characteristics area by area.

Particularly, and according to an aspect of the invention, at least one of these materials, once printed, have decreased rigidity characteristics in regards to the others so as to provide an immobilization device with decreased rigidity areas.

Advantageously, therefore, the invention's method allows providing personalized tutors in which are present and well defined areas with different rigidity between them.

According to another aspect, at least one of the materials is electrically conductive so as to achieve, in the immobilization device, areas where are present electrodes for the proper execution of the electromagnetic treatments.

Still advantageously, therefore, since the resulting device from the invention's method incorporated within itself all the necessary electrodes to the several electromagnetic treatments suitable for the correct functional recovery of the affected body segment, it allows optimizing the recovery process. Particularly, not only all the electrodes are already available, but are also positioned in the most suitable points and are not affected by the immobilization device shielding.

In addition, still advantageously, the execution of the electrodes is simultaneous to the execution of the immobilization device due to the presence of nozzles for that effect in the 3D printer. This does not only simplify the execution of the invention's device, but allows also the electrode's best arrangement and integration.

As for all of the abovementioned it is evident that the previous objects of the invention are achieved also by an immobilization device applicable to a body segment of a patient to at least partially immobilize it, comprising a shaped device according to the body shape of the patient, and in which one or more areas comprises electrically conductive material for the proper performance of electromagnetic treatments, said shaped device being printed by a 3D printer provided with at least two printer nozzle supplied at least one of which provided by said electrically conductive material.

It is also evident that the abovementioned objects are achieved by an immobilization device, applicable to at least one body segment of a patient to at least partially immobilize it, comprising a shaped body according to the body segment of the patient and in which there are one or more areas comprising decreased rigidity materials for the execution of softer areas, said shaped device being printed by a 3D printer provided with at least two printer nozzle at least one of which supplied with said decreased rigidity material.

### Brief description of the drawings

Further features and advantages of the invention will appear more evident by reading the detailed description of some preferred, but not exclusive, embodiments of a building method of an immobilization device applicable to at least one body segment of a patient to at least partially immobilize it, shown as non-limitative example with the help of the annexed drawings, wherein:

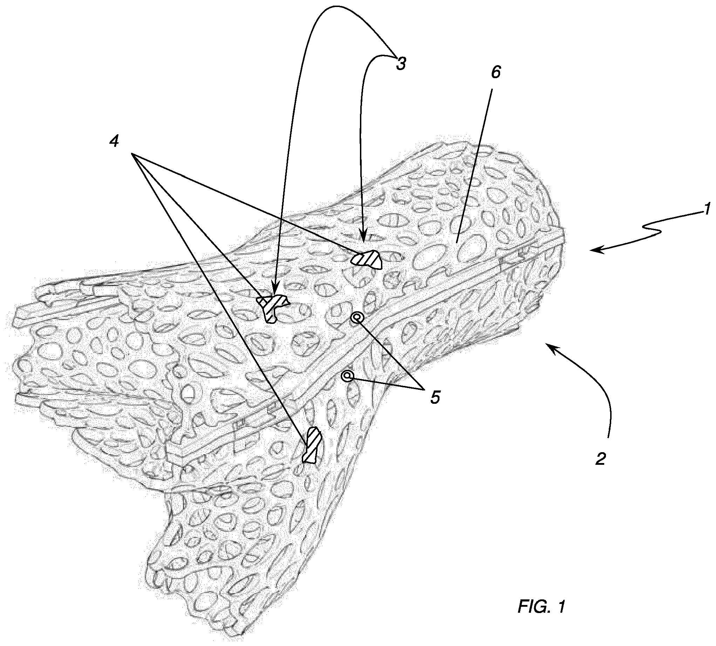

FIG. 1 represents an immobilization device according the invention in axonometric view;

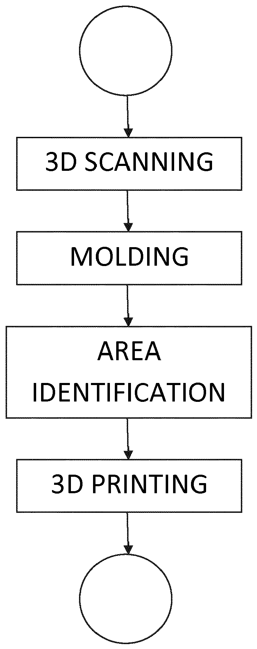

Fig. 2 represents the invention's method with a flowchart.

### Detailed description of an example of a preferred embodiment

With reference to the figures, it is described a building method of an immobilization device 1 applicable to a body segment of a patient to at least partially immobilize it.

It is observed in the figures a device 1 suited to immobilize an hand, but such use is not to be considered limitative for different embodiments of the invention, as the immobilization device of the invention acts to at least partially immobilize a shoulder, an arm, a wrist, one or more fingers, a leg, a foot, or the torso.

According to a feature of the invention, the method comprises a detection step of the volumetric dimensions of the affected patient's body segment. This step is usually performed by a 3D scan of that body segment.

With such scanning will then proceed to reconstruct the shape of the immobilization device **1.** This shaping is determined according to multiple factors. First of all, there is the volumetric dimension of the scanned body segment. However there are other factors involved in the shaping, such as, for example, the most correct posture to be imposed to the body segment to be immobilized. It is still often necessary to provide an enlargement percentage for a possible swelling, especially in cases of contusions, fractures or surgical procedures.

Thereafter there is the immobilization device **1** execution step that, according to a feature of the invention, is performed by 3D printing.

Advantageously, therefore, the device is not done manually, but by a specific machine that allows a more precise and not handmade execution. Particularly, the immobilization device 1 is not affected by a possible lack of execution expertise by the operator.

However, still advantageously, the abovementioned stages allows the operator to perform the immobilization device **1** optimizing it according to the patient's body segment, on its conditions, and according to the most correct posture to assume. In other words, advantageously, the immobilization device 1 does not result merely from an industrial production, but is performed through the described process, so that it is possible to combine the advantages of a handmade execution of an immobilization device (perfect fit to the patient) to the advantages of a mechanical execution (accuracy of the immobilization device and independence from execution error's done by the operator).

According to another aspect of the invention, the 3D printer comprises a pair of nozzles for the emission of materials that have different physical characteristics between them, in order to vary, area by area, the physical characteristics of the immobilization device **1.**

Advantageously, as said, this allows customizing the tutor during its execution, not only adapting it perfectly to the specific postural needs, but also changing the physical characteristics area by area. Particularly, such freedom to act by the operator allows also aesthetic customizations.

More in detail, according to another aspect of the invention, one of the available nozzles is used to extrude a material that, once printed presents decreased rigidity characteristics in regards to other extruded materials with other nozzles. This allows creating decreased rigidity areas.

In other words, the type of immobilization device must not be considered limitative for the invention. In fact, the device **1** can be rigid, partially flexible, or completely flexible. Particularly, each part of the device can has different rigidity degrees from each other in order to make it more suitable to the held therapeutic treatment.

It was abovementioned that the number of nozzles of the 3D printer is two, but obviously that characteristic must not be considered limitative for the invention.What is important is that the 3D printer can extrude two or more different materials simultaneously, in order to obtain different physical characteristic areas.

As a further example, according to some embodiments some areas are performed with transparent materials to the electromagnetic radiations used in imaging diagnosis, while other are performed intentionally with shielding materials.

It was previously mentioned that the immobilization devices may constitute an obstacle for the rehabilitative treatments execution, particularly to electromagnetic treatments for which the device can act as a shield (as in Magnetotherapy or TECARtherapy treatments) or an insuperable obstacle (as in case of electrostimulation via TENS).

For this reason the method of the invention provides, before the 3D printing step of the device **1** of the invention, a step in which determining the patient's body segment areas where the electrodes for the rehabilitative electromagnetic treatments, as the ones mentioned, should be arranged. Consequently, in this step they are determined the areas **3** of the immobilization device **1** where the electrodes **4** should be located for the proper performance of all the electromagnetic treatments necessary or useful to the patient's care.

For that, according to another feature of the invention, the 3D printer has a nozzle to emit electric conductive material in correspondence to the determined areas 3 so to create the electrodes **4.**

The specific area determination can be left to the operator that manually defines by the control unit with which it is executed the described method. However, that should not be considered limitative for different embodiments of the invention, according to which the control unit, once reported the needed type of rehabilitation, autonomously provides to identify those areas.

Advantageously, therefore, the immobilization device **1** comprises all the needed electrodes **4** for the electromagnetic treatments which the patient should undergo. This not only makes the performance of these treatments handier, but in certain cases (as in TENS and TECARtherapy treatments) makes them possible, on the contrary to what occurs with the known equivalent immobilization devices.

Still advantageously, since the said electrodes **4** are integral part of the immobilization device **1** of the invention, this does not constitute an obstacle to the treatments.

Still advantageously, the 3D printing of the invention device **1** allows to perform the electrodes **4** simultaneously to the device **1** with substantially unchanged costs compared to a device devoid of those electrodes.

In case of electrostimulation treatments, two or more areas **3** comprise contact points with the body segment of the patient. In cases of magnetic treatments, as some types of Magnetotherapy, other areas **3** comprise substantially laminar objects made with electrical conductive material and embedded in an electrically insulating material. These act as antennas for the magnetic field generation susceptible to work on the patient's body segment in the most suitable points.

These electrodes **4,** moreover, must be opportunely electrically powered. For this reason, and according to another aspect of the invention, each one of the areas **3** are operatively connected to corresponding electrical contact points **5** arranged on the outer surface **6** of the immobilization device **1.**

It is evident that the number of contact point **5** is not a limitative characteristic for the invention.What is observed, however, is that both these electrical contact points **5** as their electrical connection to the electrodes **4** are easily advantageously made during the 3D printing process and operated by the nozzle supplied with the electrically conductive material.

Regarding the shaping determination step of the immobilization device **1,** as previously mentioned it is performed usually by a central unit which considers several parameters, among which the shape of the affected body segment, the posture to be maintained and the possible swelling. However, it was previously said that the device **1** must not be necessarily totally rigid, but in certain parts may be variably soft.

In order to increase the tutor's possible gradation of rigidity area by area and point by point, and above all, to grade the passage between areas with decreased rigidities, there is also a calculation step of the thickness, point by point, according to the rigidity coefficient to be obtained in the shaping step of the device **1.** This allows optimizing either the behavior of the device **1** as its suitability.

Since, as said, the same immobilization device **1** comprises areas **3** where are placed the electrodes **4,** the thickness calculation step comprises, for those areas **3,** the thickness calculation of either the electrical conductive material and the electrical insulating material.

As for all the abovementioned, it is evident that object of the invention are also the immobilization devices **1** obtained with the described method.

In this regard, object of the invention is an immobilization device comprising a shaped body **2,** whose shape is in accordance with the patient's body segment to immobilize.

In the shaped body **2** there can be identified one or more areas **3** comprising electrically conductive material to make the electrodes **4** for the proper performance of the electromagnetic treatments.

The shaped body 2 is made by using a 3D printer provided with at least two printer nozzles, at least one of which supplied with electrically conductive material.

Likewise, object of the invention is also an immobilization device comprising the shaped body **2** where are identified one or more areas comprising decreased rigidity material to create softer areas.

In that case the shaped body is printed by a 3D printer provided with at least two nozzles, at least one of which is supplied with decreased rigidity material.

It is omitted here a complete discussion of the immobilization devices **1** previously mentioned, since it is repetitive as for said made for the production method description.

For all the abovementioned, it is evident that the building method of an immobilization device applicable to at least one body segment of a patient to at least partially immobilize it, as well as the devices themselves, achieves all the intended purposes.

Particularly, the obtained immobilization devices have areas with differenced rigidities and its execution results easy, fast and accurate.

The same devices allow easy application of therapies such as TENS, Magnetic or Tecartherapy without having to remove them.

The invention is susceptible to several modifications e variations, all falling within the inventive concept expressed in the claims. All the details may be replaced by other technically equivalent elements, and the materials may be different according to requirements, without departing the scope of the invention defined by the appended claims.

## Claims

1. A building method of an immobilization device **(1)** applicable to at least one body segment of a patient for at least partially immobilize it, said method comprising the following steps:
- detecting the volumetric dimensions of the patient's body segment;
- setting the shaping of said immobilization device **(1),**
said method being **characterized in** comprising a step of printing said immobilization device **(1)** using a 3D printer, said 3D printer comprising at least one pair of nozzles for the emission of materials having different physical characteristics so as to vary, area by area, the physical characteristics of said device immobilization **(1).**

2. Method according to claim 1, **wherein** at least one of said materials, once printed, have rigidity characteristics decreased compared to the other so as to obtain, in said immobilization device **(1),** areas with decreased rigidity.

3. Method according to claim 1 or 2, **wherein** at least one of said materials is an electrically conductive material so as to obtain, in said immobilization device **(1),** areas **(3)** in which are present electrodes **(4)** for the correct execution of electromagnetic treatments.

4. Method according to claim 3, **wherein** said method comprises, before said 3D printing step, a determining step of the areas **(3)** where must be placed said electrodes **(4)** for the execution of electrostimulation treatments.

5. Method according to any one of the preceding claims, **wherein** said determining step of the shape of said immobilization device **(1)** comprises a calculation step of the thicknesses of said immobilization device **(1)** based on the stiffness coefficient that must have said immobilization device **(1)** on each point of the body segment.

6. Method according to any one of the preceding claims, **wherein** said calculating step of the thickness of said immobilization device **(1)** comprises, for said areas **(3)** where are present said electrodes **(4),** the calculation of the thickness of the portion made by electrically conductive material and the thickness of the portion made by electrically insulating material.

## Patentansprüche

1. Herstellungsverfahren einer Immobilisierungsvorrichtung (1), die auf mindestens ein Körpersegment eines Patienten angewandt werden kann, um ihn mindestens teilweise zu immobilisieren, wobei das Verfahren die folgenden Schritte umfasst:
- Detektieren der volumetrischen Abmessungen des Körpersegments des Patienten;
- Einstellen der Formgebung der Immobilisierungsvorrichtung (1),
wobei das Verfahren **dadurch gekennzeichnet ist, dass** es einen Schritt des Druckens der Immobilisierungsvorrichtung (1) unter Verwendung eines 3D-Druckers umfasst, wobei der 3D-Drucker mindestens ein Düsenpaar zur Emission von Materialien mit unterschiedlichen physikalischen Eigenschaften umfasst, um die physikalischen Eigenschaften der Immobilisierungsvorrichtung (1) flächenweise zu variieren.

2. Verfahren nach Anspruch 1, wobei mindestens eines der Materialien nach dem Drucken im Vergleich zu den anderen geringere Steifheitseigenschaften aufweist, um in der Immobilisierungsvorrichtung (1) Bereiche mit verringerter Steifigkeit zu erhalten.

3. Verfahren nach Anspruch 1 oder 2, wobei mindestens eines der Materialien ein elektrisch leitendes Material ist, um in der Immobilisierungsvorrichtung (1) Bereiche (3) zu erhalten, in denen Elektroden (4) für die korrekten Durchführung elektromagnetischer Behandlungen vorhanden sind.

4. Verfahren nach Anspruch 3, wobei das Verfahren vor dem 3D-Druckschritt einen Bestimmungsschritt der Bereiche (3), in denen die Elektroden (4) zur Durchführung von Elektrostimulationsbehandlungen angeordnet sein müssen, umfasst.

5. Verfahren nach einem der vorhergehenden Ansprüche, wobei der Bestimmungsschritt der Form der Immobilisierungsvorrichtung (1) einen Berechnungsschritt der Dicken der Immobilisierungsvorrichtung (1) basierend auf dem Steifheitskoeffizienten, den die Immobilisierungsvorrichtung (1) an jedem Punkt des Körpersegments aufweisen muss, umfasst.

6. Verfahren nach einem der vorhergehenden Ansprüche, wobei der Berechnungsschritt der Dicke der Immobilisierungsvorrichtung (1) für die Bereiche (3), in denen die Elektroden (4) vorhanden sind, die Berechnung der Dicke des Teils aus elektrisch leitendem Material und der Dicke des Teils aus elektrisch isolierendem Material, umfasst.

## Revendications

1. Procédé de construction d'un dispositif d'immobilisation **(1)** applicable à au moins un segment de corps d'un patient pour au moins partiellement l'immobiliser, ledit procédé comprenant les étapes suivantes :
- la détection des dimensions volumétriques du segment de corps du patient ;
- la définition de la mise en forme dudit dispositif d'immobilisation **(1),**
- ledit procédé étant **caractérisé en ce qu'**il comprend une étape d'impression dudit dispositif d'immobilisation **(1)** en utilisant une imprimante 3D, ladite imprimante 3D comprenant au moins une paire de buses pour l'émission de matériaux présentant différentes caractéristiques physiques pour faire varier, zone par zone, les caractéristiques physiques dudit dispositif d'immobilisation **(1).**

2. Procédé selon la revendication 1, **dans lequel** au moins l'un desdits matériaux, une fois imprimé, présente des caractéristiques de rigidité réduites comparé à l'autre de façon à obtenir, dans ledit dispositif d'immobilisation **(1),** des zones avec une rigidité réduite.

3. Procédé selon la revendication 1 ou 2, **dans lequel** au moins l'un desdits matériaux est un matériau électroconducteur pour obtenir, dans ledit dispositif d'immobilisation **(1),** des zones **(3)** dans lesquelles des électrodes **(4)** sont présentes, pour l'exécution correcte de traitements électromagnétiques.

4. Procédé selon la revendication 3, **dans lequel** ledit procédé comprend, avant ladite étape d'impression 3D, une étape de détermination des zones **(3)** où lesdites électrodes **(4)** doivent être placées pour l'exécution des traitements d'électrostimulation.

5. Procédé selon l'une quelconque des revendications précédentes, **dans lequel** ladite étape de détermination de la forme dudit dispositif d'immobilisation **(1)** comprend une étape de calcul de l'épaisseur dudit dispositif d'immobilisation **(1)** basée sur le coefficient de rigidité que ledit dispositif d'immobilisation **(1)** doit présenter sur chaque point du segment de corps.

6. Procédé selon l'une quelconque des revendications précédentes, **dans lequel** ladite étape de calcul de l'épaisseur dudit dispositif d'immobilisation **(1)** comprend, pour lesdites zones **(3)** dans lesquelles sont présentes lesdites électrodes **(4),** le calcul de l'épaisseur de la partie formée par le matériau électroconducteur et de l'épaisseur de la partie formée par le matériau électriquement isolant.
